# EUROPEAN PATENT SPECIFICATION

(11) **EP 4 125 924 B1**
(45) Date of publication and mention of the grant of the patent: **24.09.2025**
(21) Application number: 21778847.0
(22) Date of filing: 31.03.2021
(51) Int. Cl.: A61K 31/341, A61K 31/635, A61K 9/00, A61K 9/72, A61P 11/00, A61P 31/14, C07D 307/52

(54) **FUROSEMIDE COMPOSITIONS FOR USE IN SUPPORTIVE THERAPY IN CORONAVIRUS INFECTION**
FUROSEMIDZUSAMMENSETZUNGEN ZUR VERWENDUNG IN DER UNTERSTÜTZENDEN THERAPIE BEI CORONAVIRUSINFEKTION
COMPOSITIONS DE FUROSÉMIDE POUR UTILISATION DANS LA THÉRAPIE DE SOUTIEN D'UNE INFECTION À CORONAVIRUS

(30) Priority: 31.03.2020 US 202063002660 P
(43) Date of publication of application: 08.02.2023
(73) Proprietor: University Health Network, Toronto, Ontario M5G 2C4 (CA)
(72) Inventor: WEAVER, Donald Fredric, Toronto, Ontario M4K 1X4 (CA); BARDEN, Christopher James, Toronto, ON M6P 2T4 (CA); REED, Mark Andrew, Puslinch, ON N0B 2J0 (CA); WANG, Zhiyu, Toronto, ON M2K 2W4 (CA)
(74) Representative: Bardehle Pagenberg Partnerschaft mbB Patentanwälte Rechtsanwälte
(86) International application number: PCT/CA2021/050438
(87) International publication number: WO 2021/195777

(56) References cited:
- US-A- 4 908 382
- US-A1- 2018 243 257
- MEHTA PUJA ET AL: "COVID-19: consider cytokine storm syndromes and immunosuppression", THE LANCET, ELSEVIER, AMSTERDAM, NL, vol. 395, no. 10229, 16 March 2020 (2020-03-16), pages 1033 - 1034, XP086105444, ISSN: 0140-6736, [retrieved on 20200316], DOI: 10.1016/S0140-6736(20)30628-0
- RUSSELL BETH ET AL: "Associations between immune-suppressive and stimulating drugs and novel COVID-19-a systematic review of current evidence", ECANCERMEDICALSCIENCE, vol. 14, 27 March 2020 (2020-03-27), XP055863895, DOI: 10.3332/ecancer.2020.1022
- WANG ET AL: "Up-regulation of IL-6 and TNF-@a induced by SARS-coronavirus spike protein in murine macrophages via NF-@kB pathway", VIRUS RESEARCH, AMSTERDAM, NL, vol. 128, no. 1-2, 11 August 2007 (2007-08-11), pages 1 - 8, XP022193892, ISSN: 0168-1702, DOI: 10.1016/J.VIRUSRES.2007.02.007
- PRANDOTA JOSEPH: "Furosemide: progress in understanding its diuretic, anti-inflammatory, and bronchodilating mechanism of action, and use in the treatment of respiratory tract diseases", AMERICAN JOURNAL OF THERAPEUTICS, LIPPINCOTT WILLIAMS & WILKINS, US, vol. 9, no. 4, 1 July 2002 (2002-07-01), pages 317 - 328, XP009104197, ISSN: 1075-2765, DOI: 10.1097/00045391-200207000-00009
- YUENGSRIGUL A ET AL: "Immunosuppressive and cytotoxic effects of furosemide on human peripheral blood mononuclear cells", ANNALS OF ALLERGY, ASTHMA, ELSEVIER, AMSTERDAM, NL, vol. 83, no. 6, 1 December 1999 (1999-12-01), pages 559 - 566, XP026958451, ISSN: 1081-1206, [retrieved on 19991201]
- ANONYMOUS: "NCT04588792: Furosemide as Supportive Therapy for COVID-19 Respiratory Failure (FaST-1)", CLINICALTRIALS.GOV, 11 December 2023 (2023-12-11), pages 1 - 8, XP093137065, Retrieved from the Internet <URL:https://classic.clinicaltrials.gov/ct2/show/NCT04588792?term=furosemide&cond=covid&draw=2&rank=1> [retrieved on 20240301]
- BRENNECKE ANJA ET AL: "Is Inhaled Furosemide a Potential Therapeutic for COVID-19?", AMERICAN JOURNAL OF MEDICAL SCIENCES, vol. 360, no. 3, 1 June 2020 (2020-06-01), USA, pages 216 - 221, XP093137021, ISSN: 0002-9629, Retrieved from the Internet <URL:https://www.ncbi.nlm.nih.gov/pmc/articles/PMC7833957/pdf/main.pdf> DOI: 10.1016/j.amjms.2020.05.044
- MUSCEDERE JOHN ET AL: "Nebulized Furosemide for Pulmonary Inflammation in Intubated Patients With COVID-19: A Phase 2 Randomized Controlled Double-Blind Study", CRITICAL CARE EXPLORATIONS, vol. 6, no. 2, 1 February 2024 (2024-02-01), pages e1045, XP093259848, ISSN: 2639-8028, Retrieved from the Internet <URL:https://pmc.ncbi.nlm.nih.gov/articles/PMC10954058/> DOI: 10.1097/CCE.0000000000001045
- WANG ZHIYU, WANG YANFEI, VILEKAR PRACHI, YANG SEUNG-PIL, GUPTA MAYURI, OH MYONG IN, MEEK AUTUMN, DOYLE LISA, VILLAR LAURA, BRENNEC: "Small molecule therapeutics for COVID-19: repurposing of inhaled furosemide", PEERJ, vol. 8, 2020, pages 1 - 21, XP055926106, DOI: 10.7717/peerj.9533
- CHAUDHRY ZAIN, SHAWE-TAYLOR MARIANNE, RAMPLING TOMMY, CUTFIELD TIM, BIDWELL GABRIELLA, CHAN XIN HUI S., LAST ANNA, WILLIAMS BRYAN,: "Short durations of corticosteroids for hospitalised COVID-19 patients are associated with a high readmission rate", JOURNAL OF INFECTION, vol. 82, no. 6, June 2021 (2021-06-01), pages 276 - 316, XP055928023, DOI: 10.1016/j.jinf. 2021.02.00 8
- MARTIN GREG S., MOSS MARC, WHEELER ARTHUR P., MEALER MEREDITH, MORRIS JOHN A., BERNARD GORDON R.: "A randomized, controlled trial of furosemide with or without albumin in hypoproteinemic patients with acute lung injury", CRITICAL CARE MEDICINE, vol. 33, no. 8, 2005, pages 1681 - 1687, XP055928026

## Description

### CROSS-REFERENCE TO RELATED APPLICATIONS

The present application claims priority from U.S. Provisional Patent Application No. 63/002,660, filed March 31, 2020.

### FIELD OF THE INVENTION

The present application pertains to the field of furosemide and pharmaceutical compositions thereof. More particularly, the present application relates to furosemide compositions useful for supportive therapy in infections caused by coronaviruses, still more particularly SARS-CoV-2. The present invention relates to furosemide, or a pharmaceutically acceptable salt, solvate or hydrate thereof, for use in the treatment of a condition associated with coronavirus infection, wherein the condition is acute respiratory distress, lung inflammation, systemic inflammation, cytokine storm, or a combination thereof.

### INTRODUCTION

The novel coronavirus SARS-CoV-2, which causes COVID-19, has become a dangerous, worldwide pathogen over the last four months, stressing health systems and ushering in a level of global societal upheaval not seen since 2008 (and arguably, since 1957's global flu pandemic).

While the first antiviral therapeutics are still at the planning stage, compelling data are developing around interleukin (and other pro-inflammatory cytokine) inhibition; these observations have resulted in a role being proposed for Roche's tocilizumab (Actemra) and Sanofi/Regeneron's sarilumab (Kevzara). Of the various interleukins, IL-6 may play a key role in driving the inflammatory response that leads to morbidity and mortality in patients with COVID-19 who develop acute respiratory distress syndrome. Arising from various sources, there is a growing body of reports showing the benefit of targeting the IL-6 pathway in patients with COVID-19, most notably from a recent single-arm study in China.

For example, there have been reports of increasing experience using tocilizumab, an IL-6 inhibitor, to treat patients with severe COVID-19 infection, including a non-peer-reviewed retrospective Chinese experience describing 21 COVID-19 positive patients with severe illness who received tocilizumab. They observed improvement in oxygenation and other clinical outcomes. In this Chinese study, conducted by Xiaolong Xu, of the First Affiliated Hospital of the University of Science and Technology of China, and colleagues, tocilizumab was given to 21 patients with severe COVID-19, between Feb. 5 and Feb. 14, 2020. According to the researchers, fevers returned to normal and all other symptoms "improved remarkably" within a few days. Additionally, 75% of patients had lowered their oxygen intake and one patient no longer needed oxygen therapy. There were no obvious adverse reactions. Nineteen patients were discharged after an average of 13.5 days following treatment, with the remainder "recovering well" as of the time of the study's release.

Concomitantly, Sanofi/Regeneron are starting phase II/III trials with their anti-IL-6 MAb, sarilumab. Sarilumab is a fully human, monoclonal antibody that inhibits the interleukin-6 pathway by binding and blocking the IL-6 receptor. According to the pharmaceutical companies' joint statement, IL-6 may play a role in driving the overactive inflammatory response in the lungs of patients who are severely or critically ill with COVID-19. Mehta Puja et al. disclose in The Lancet, 16.03.2020, p. 1033-1034, that a multi-centre, randomised controlled trial of tocilizumab (IL-6 receptor blockade), has been approved in patients with COVID-19 pneumonia and elevated IL-6 in China. Russell Beth et al. disclose in Ecancermedicalscience, 27.03.2020, p. 1-43, that there is evidence that IL-6 peak levels are associated with severity of pulmonary complications of Covid-19; however, there is no scientific evidence of the beneficial impact of IL-6 inhibitors in the modulation of the COVID-19 infection. Wang et al. disclose in Virus Research, 2007, p. 1-8, that the interaction between SARS-CoV spike (S) protein and murine macrophages induces cytokines IL-6 and TNF-alpha release.

In addition to targeting IL-6, other cytokines are also being considered. For example, trials using adalimumab, which targets TNF-α, have also been proposed. However, clearly the side-effects of such biologic drugs - sarilumab and tocilizumab - carry black box warnings over their risk of predisposing to serious infections in patients with pulmonary disease, and thus their use will need to be closely monitored.

Moreover, although these biologics are potent therapeutics, they do have significant issues when applied to the problem of COVID-19: (1) They may be "too specific"; accordingly, a broad spectrum agent targeting more than one cytokine may be of greater value, since COVID-19 infection is associated with elevation of multiple pro-inflammatory cytokines; (2) They are administered systemically (rather than locally to the lungs) and thus may produce systemic side-effects, including secondary infections, unwanted in a severely ill person; and (3) They are expensive to produce, resulting in shortages in first world countries and complete non-availability in developing countries.

Accordingly, small molecule therapeutics, particularly compounds that have a history of clinical safety and that are widely available, would have improved value over biologics. There remains a need to identify and evaluate such compounds. A need also remains for new compounds or agents for therapeutic regimes to arrest or reverse respiratory distress, air hunger, shortness of breath, inflammation, cytokine storm, and associated symptoms or conditions that accompany coronavirus infection.

This application is co-pending with "Furosemide analogues and compositions and uses thereof for Alzheimer's disease", International PCT Publication No. WO 2020/257940, filed June 26, 2020, based on U.S. Provisional Patent Application Ser. No. 62/866,735, filed June 26, 2019; and U.S. Provisional Patent Application Ser. No. 62/985,547, filed March 5, 2020.

### SUMMARY

An object of the present application is to provide compounds and compositions useful in preventing, delaying, or treating the respiratory symptoms and conditions associated with coronavirus infection.

In accordance with an aspect of the present invention, there is provided furosemide: 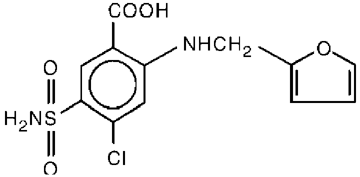 or a pharmaceutically acceptable salt, solvate or hydrate thereof, for use in the treatment of a condition associated with coronavirus infection, wherein the condition is acute respiratory distress, lung inflammation, systemic inflammation, cytokine storm, or a combination thereof.

### BRIEF DESCRIPTION OF THE FIGURES

For a better understanding of the application as described herein, as well as other aspects and further features thereof, reference is made to the following description which is to be used in conjunction with the accompanying drawings.

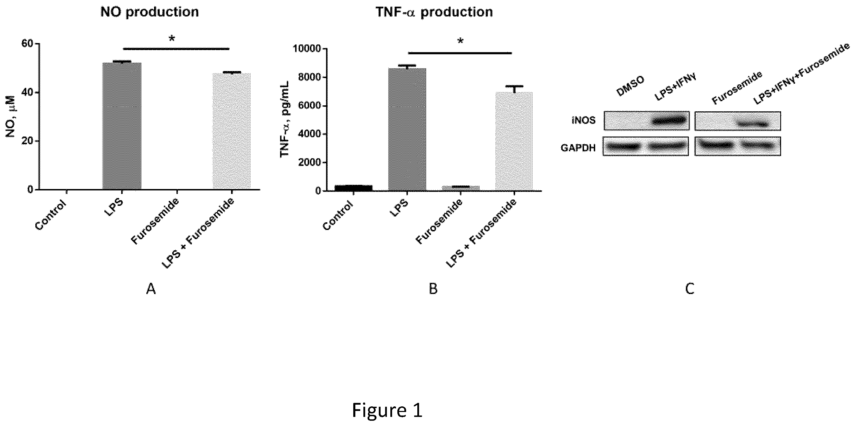

Figure 1: Furosemide decreases the production of NO and TNF-α. Production of (A) NO and (B) TNF-α from RAW264.7 cells upon LPS induction was determined by Griess assay and ELISA from the conditioned medium. Error bars show SEM, *n* = 3. *, *p* < 0.05. (C) iNOS expression level from RAW264.7 cells was assessed by Western blot analysis. Cells were treated with LPS+IFNγ with DMSO or 25 µM furosemide. After 24 h of incubation, cell lysate was harvested for Western blot analysis. GAPDH, Glyceraldehyde 3-phosphate dehydrogenase.

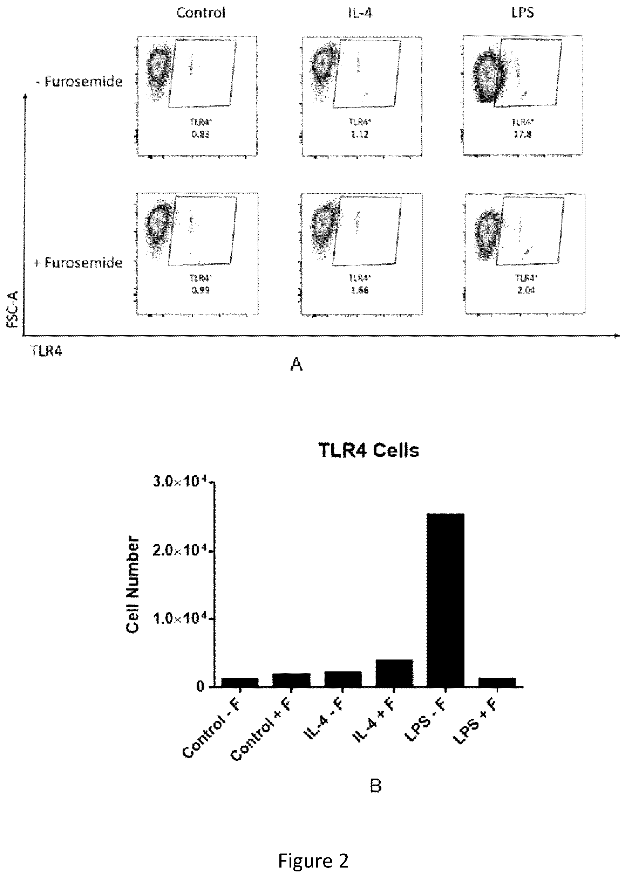

Figure 2: Furosemide significantly decreases TLR4+ cell population. RAW264.7 macrophage cells were stimulated with LPS and flow cytometry was used to determine TLR4+ cells population. (A) % of Vis and (B) cell numbers for TLR4+. F, furosemide, FSC-A, front scatter.

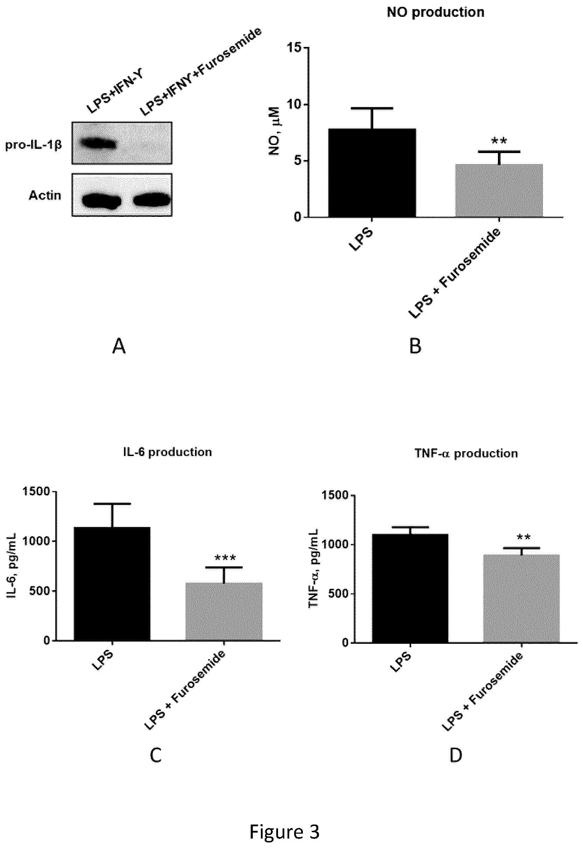

Figure 3: Furosemide significantly inhibits pro-inflammatory responses. Furosemide activity was initially tested on differentiated THP-1 macrophages. THP-1 monocytic cells were initially differentiated to THP-1 macrophages by PMA for 24 h. Cells were then stimulated by LPS/IFNy in the presence of furosemide or DMSO control. Followed by 48 h incubation, the conditioned media and cell lysates were harvested for analysis. (A) Western blot showing the expression of pro-IL-1β upon treatment with DMSO and furosemide, respectively. Actin was used as a loading control. Productions of (B) NO, (C) IL-6 and (D) TNF-α from SIM-A9 cells were measured by either Griess assay or ELISA. Error bars show SD, *n =* 6. **, *p* < 0.01; ***, *p* < 0.001.

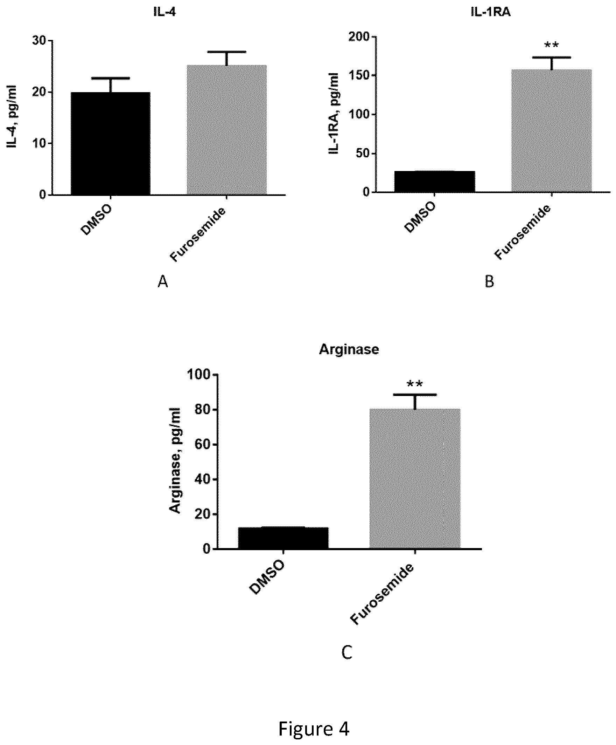

Figure 4: Furosemide induces the expression of anti-inflammatory phenotype markers on THP-1 macrophages. The anti-inflammatory activity of furosemide was tested by THP-1 macrophages. THP-1 monocytic cells were initially differentiated to THP-1 macrophage by PMA for 24 h. Cells were then stimulated by LPS/IFNy in the presence of furosemide or DMSO control. Followed by 48 h incubation, the conditioned media were harvested for (A) IL-4, (B) IL-1RA and (C) Arginase analysis. Error bars show SD, *n =* 2. ***, p <* 0.01.

### DETAILED DESCRIPTION

### Definitions

Unless defined otherwise, all technical and scientific terms used herein have the same meaning as commonly understood by one of ordinary skill in the art to which this invention belongs.

As used in the specification and claims, the singular forms "a", "an" and "the" include plural references unless the context clearly dictates otherwise.

The term "comprising" as used herein will be understood to mean that the list following is non-exhaustive and may or may not include any other additional suitable items, for example one or more further feature(s), component(s) and/or ingredient(s) as appropriate.

The term "subject" is used herein interchangeably with the term "patient" to refer to a mammal, such as a human, in need of treatment or potentially in need of treatment.

The terms "treatment" or "treating," as used herein, mean an approach for obtaining beneficial or desired results, including clinical results. Beneficial or desired clinical results can include alleviation or amelioration of one or more symptoms or conditions, diminishment of extent of disease, stabilized (i.e., not worsening) state of disease, preventing spread of disease, delay or slowing of disease progression, amelioration or palliation of the disease state, and remission (whether partial or total), whether detectable or undetectable.

The term "therapeutically effective amount," "effective amount" or "sufficient amount" of a compound of the present application is a quantity sufficient to, when administered to the subject, including a mammal, for example a human, effect beneficial or desired results, including clinical results, and, as such, an "effective amount" or synonym thereto depends upon the context in which it is being applied.

Moreover, a "treatment" or "prevention" regime of a subject with a therapeutically effective amount of an agent may consist of a single administration, or altematively comprise a series of applications. For example, the agent may be administered at least once a week. However, in another embodiment, the agent may be administered to the subject from about one time per week to about once daily for a given treatment, or multiple doses per day. The length of the treatment period depends on a variety of factors, such as the severity of the disease, the age of the patient, the concentration and the activity of the agent, or a combination thereof. It will also be appreciated that the effective dosage of the agent used for treatment or prophylaxis may increase or decrease over the course of a particular treatment or prophylaxis regime. Changes in dosage may result and become apparent by standard diagnostic assays known in the art. **In** some instances, chronic administration may be required.

The term "administering" is defined as any conventional route for administering an agent(s) to a subject for use as is known to one skilled in the art. This may include, for example, administration via the parenteral (i.e., inhalation, subcutaneous, intradermal, intramuscular, etc.) or mucosal surface route. **In** other embodiments this may include oral administration. The dose of the agent(s) may vary according to factors such as the health, age, weight and sex of the animal. The dosage regime may be adjusted to provide the optimum dose. One skilled in the art will appreciate that the dosage regime can be determined and/or optimized without undue experimentation.

To "inhibit" or "suppress" or "lower" or "reduce" or "down regulate" a function or activity, is to reduce the function or activity when compared to otherwise same conditions except for a condition or parameter of interest, or alternatively, as compared to another condition or control.

The present application provides compounds and compositions for use in preventing, delaying, or treating the respiratory symptoms and conditions associated with coronavirus infection: notably, furosemide (4-chloro-N-furfuryl-5-sulfamoylanthranilic acid). Furosemide is a safe, inexpensive, well-studied small molecule with a long history of industrial synthesis (see for example U.S. Patent 5,739,361, Example 3).

While no one has previously considered the use of furosemide in subjects infected with a coronavirus, there is a history of experimental use of furosemide for respiratory conditions.

More than twenty years ago, the concept of inhaled diuretics, like furosemide, was explored as an approach to reduce dyspnea, primarily from the rationale that swollen, edematous airway mast cells would be reduced in size following diuresis. However, further investigations have established that mechanism of action is not related to local diuretic effect or engagement of the Na⁺/K⁺-ATP shuttle (indeed, patients receiving inhaled furosemide up to 40 mg report no increase in urge to urinate). Irrespective of its mechanism of action, multiple clinical investigations have reported reduction in lung IL-6, IL-8, and TNF-α levels upon administering inhaled furosemide to patients with respiratory conditions, including in neonatal tachypnoea (NCT01407848), bronchopulmonary dysplasia (NCT03946891), COPD, and dyspnea and breathlessness broadly (NCT02881866, NCT02524054, NCT01851980, NCT01440764). Clinically, relief of air hunger is broadly reported, including in steroid-dependent asthma, and in hypercapnia-induced healthy controls in a randomized controlled trial (NCT04130815).

In some embodiments, dosing is oral and is given 40 mg per day. In some embodiments, dosing is oral and is given 80 mg/day via 40 mg p.o., twice daily. In some embodiments, dosing is intravenous and is given bolus. In some embodiments, dosing is intravenous and is given by infusion.

Dosing in previous studies has shown safety up to 120 mg per dose by inhalation, and it is likely that higher doses are also tolerated. Studies examining at repeat-dose administration suggest that additional relief is provided over single dosing and is well tolerated. In some embodiments, dosing is inhaled and is given by nebulization, 40 mg/dose. In some embodiments, dosing is inhaled and is given by nebulization, 20 mg/dose. In some embodiments, nebulized dosing is given once per day, or twice per day, or three times per day, or four times per day, or more often according to a physician's judgment. In some embodiments, dosing is adjusted to appropriate mg/kg amounts based on the weight of the subject.

The role of IL-6 as a potential inflammatory target for avoiding "cytokine storm" and further deterioration and/or mortality of COVID-19 patients is further support for exploring whether furosemide could be of value. It is especially important to consider furosemide in this context given that, while in Canada and other industrialized nations, biologics against IL-6 might be available, this is not the case in other nations impacted by the pandemic. There would be a high community/public benefit for provision of a small molecule that is inexpensive and widely available to combat COVID-19 morbidities.

The present inventors have shown that furosemide has the ability to reduce the production of pro-inflammatory cytokines (e.g., from macrophages in cellular models). See the Examples given in the Applicant's co-pending PCT patent application (WO 2020/257940). The present inventors have further found that upon treatment with furosemide, LPS-induced production of pro-inflammatory cytokines was reduced, indicating that furosemide suppresses the M1 polarization, including IL-6 and TNF-α release. In addition, it was found that furosemide promotes the production of anti-inflammatory cytokine products (IL-1RA, arginase), indicating M2 polarization. These results demonstrate that furosemide is a reasonably potent inhibitor of IL-6 and TNF-α that is also safe, inexpensive, and well-studied.

Further background confirming the safe use of furosemide as an inhaled medicine can be found in the following:
Grogono JC, Butler C, Izadi H, Moosavi SH. Inhaled furosemide for relief of air hunger versus sense of breathing effort: a randomized controlled trial. Respir Res. 2018 Sep 20;19(1):181. doi: 10.1186/s12931-018-0886-9. PubMed PMID:30236110; PubMed Central PMCID: PMC6148783.
Inokuchi R, Aoki A, Aoki Y, Yahagi N. Effectiveness of inhaled furosemide for acute asthma exacerbation: a meta-analysis. Crit Care. 2014 Nov 24;18(6):621. doi: 10.1186/s13054-014-0621-y. PubMed PMID: 25673428; PubMed Central PMCID:PMC4241398.
Murad H, Ghabrah T, Rafeeq M, Ali S. Subdiuretic dose of furosemide enhances albuterol effects in asthmatic mice rather than bumetanide. Allergol Immunopathol (Madr). 2018 Nov - Dec;46(6):585-593. doi: 10.1016/j.aller.2018.05.001. Epub 2018 Sep 5. PubMed PMID: 30193887.
Ong KC, Kor AC, Chong WF, Earnest A, Wang YT. Effects of inhaled furosemide on exertional dyspnea in chronic obstructive pulmonary disease. Am J Respir Crit Care Med. 2004 May 1;169(9):1028-33. Epub 2004 Feb 20. PubMed PMID: 14977622. See also the data supplement. Waskiw-Ford M, Wu A, Mainra A, Marchand N, Alhuzaim A, Bourbeau J, Smith BM,
Jensen D. Effect of Inhaled Nebulized Furosemide (40 and 120 mg) on Breathlessness during Exercise in the Presence of External Thoracic Restriction in Healthy Men. Front Physiol. 2018 Feb 12;9:86. doi: 10.3389/fphys.2018.00086. eCollection 2018. PubMed PMID: 29483879; PubMed Central PMCID: PMC5816054.
Yuengsrigul A, Chin TW, Nussbaum E. Decreased Cytokine Production from Human Peripheral Blood Mononuclear Cells by Furosemide. Pediatric Research 1996; 39:15-18.
Yuengsrigul A, Chin TW, Nussbaum E. Immunosuppressive and cytotoxic effects of furosemide on human peripheral blood mononuclear cells. Ann Allergy Asthma Immunol. 1999 Dec;83(6 Pt 1):559-66. PubMed PMID: 10619350.

### Compositions

The compositions containing furosemide, or a salt, solvate or hydrate thereof, can be prepared by known methods for the preparation of pharmaceutically acceptable compositions which can be administered to subjects, such that an effective quantity of the active agent(s) is combined in a mixture with a pharmaceutically acceptable vehicle. Suitable vehicles are described, for example, in Remington's Pharmaceutical Sciences (2003 - 20th edition) and in The United States Pharmacopeia: The National Formulary (USP 24 NF19) published in 1999. On this basis, the compositions include, albeit not exclusively, solutions of the compound(s) in association with one or more pharmaceutically acceptable vehicles or diluents, and/or contained in buffered solutions with a suitable pH and iso-osmotic with the physiological fluids.

The compositions can be formulated for administration to a subject alone or in combination with pharmaceutically acceptable carriers, as noted above, and/or with other pharmaceutically active agents for preventing, delaying, or treating coronavirus infection or its symptoms, the proportion of which is determined by the solubility and chemical nature of the agents, chosen route of administration and standard pharmaceutical practice.

The dosage of the compound(s) and/or compositions can vary depending on many factors such as the pharmacodynamic properties of the agent, the mode of administration, the age, health and weight of the recipient, the nature and extent of the symptoms, the frequency of the treatment and the type of concurrent treatment, if any, and the clearance rate of the compound in the animal to be treated. One of skill in the art can determine the appropriate dosage based on the above factors. The compound(s) can be administered initially in a suitable dosage that may be adjusted as required, depending on the clinical response.

Furosemide is a commonly prescribed diuretic drug, listed on the WHO's List of "Essential Medicines" for its long history of safety and efficacy. It is commonly given orally or intravenously (bolus or infusion), but it has also been shown to be safely administered locally to the lung through inhalation; for example, via nebulization, dry powder inhalation, or metered-dose inhaler. See for example U.S. Patents 8,883,845; 6,026,809; 5,392,767. Furosemide is soluble in alkali solutions. Solutions of non-salt furosemide can be made at 10 mg/mL concentrations in aqueous isotonic sodium choride (saline), pH-adjusted with sodium hydroxide or hydrochloric acid as necessary to obtain a pH between 8.0 and 9.3. In some embodiments, furosemide is prepared in 10 mg/mL solution containing NaCl 7.0 mg, plus addition of NaOH solution to adjust to pH 9.

In some embodiments, the furosemide solution is used for administration using a nebulizer, at a dose amount of from 2 to 6 mL, or about 4 mL, of a 10 mg/mL solution, for example as described above. In certain embodiments the dose of furosemide is determined based on the patient's weight and can be, for example, from about 0.1 to about 1 mg/kg.

Previous studies suggest that furosemide is shuttled from the lung and excreted within 4-6 hours. Consequently, repeated dosing may be necessary depending on the severity of the patient's condition. In certain embodiments, administration is once daily, twice daily, three time daily or four times daily. In certain embodiments the daily dose of furosemide (or total amount of furosemide administered per day) is between about 20 mg and about 300 mg, or more particularly from about 20 mg to about 200 mg. For example, the daily dose can be about 200 mg, about 180 mg, about 160 mg, about 140 mg, about 120 mg, about 100 mg, about 80 mg, about 60 mg, about 40 mg, or about 20 mg.

To gain a better understanding of the invention described herein, the following examples are set forth. It should be understood that these examples are for illustrative purposes only. Therefore, they should not limit the scope of this invention in any way. The invention is as defined in the claims.

### EXAMPLES

### EXAMPLE 1: Clinical Study

A demonstration of efficacy of furosemide in the treatment of respiratory conditions associated with COVID-19 (SARS-CoV-2) infection can be performed according to the clinical study described herein.

Subjects will be recruited on a rolling basis from an Intensive Care Unit of a hospital. Subjects under severe respiratory distress for which intubation is indicated, who additionally have fever (defined as >36.6°C [axilla], >37.2°C [oral] or >37.8°C [rectal]) or cough (and in the opinion of the assessing physician to have presumptive COVID-19) and willing to be enrolled can be provisionally entered into the study, given a study participant number, and randomized into one of two groups by an external randomizer. The randomizer will indicate whether the study participant is treated with furosemide treatment or with control agent (placebo). Patients enrolled in the study will also have laboratory-confirmed COVID-19 (SARS-CoV-2) infection as determined by polymerase chain reaction (PCR) or other commercial or public health assay within ten (10) days of entering the trial.

A set of 40 vials will be prepared for each patient subject. Each set of 40 vials will contain either all treatment or all control agents, depending on the group to which the subject has been randomly assigned.

During the study furosemide will be administered by inhalation for up to ten days. Based on previous inhalation clinical trials of furosemide, furosemide will be prepared in 10 mg/mL solution containing NaCl 7.0 mg, plus addition of NaOH solution to adjust to pH 9.

Depending on the group assigned, subjects will receive at each dosing either:
- Treatment - 4 mL of 10 mg/mL furosemide in 0.9% saline solution
- Placebo - 4 mL of 0.9% saline solution

Each of these will be previously prepared in sterile conditions using API produced according to Good Manufacturing Practice and packaged into tamper-resistant nebulizer vials labelled with the trial number, study participant number, and vial number.

Baseline participant characteristics will be assessed and recorded prior to administration of either the intervention or placebo. Subjects will be assessed for baseline measurements of vital signs including temperature, systolic blood pressure, and pulse.

Subjects will be allowed all standard of care (SOC) for COVID-19, which for respiratory symptoms may include treatment with a bronchodilator such as albuterol. Patients who are receiving bronchodilation will be so indicated in the case report form (CRF) and subject to potential subgroup analysis.

20-30 minutes after receiving the bronchodilator (if bronchodilator is prescribed) or immediately after initial assessment (if bronchodilator is not prescribed), the first coded vial for the subject will be administered using a nebulizer over a 10-15 min period. This is Day 1, Dose 1 and will result in 40 mg furosemide being nebulized, or 4 mL of saline in the control group.

Vitals, arterial blood gases, PaO2, FiO2, extrinsic PEEP and other ventilator settings as usually collected in ICU will be recorded every six hours during the intervention period, followed by bronchodilator nebulization if indicated, 20-30 min waiting period, and the next coded vial for the subject will be nebulized. See the table below for the schedule of other tests.

Intervention will continue every six hours through vial 40 (i.e., through Day 10, Dose 4) or until the subject has either achieved step-down from ICU or has died.

If the subject is still hospitalized after completing the first 40 vials, the intervention will stop at that point and SOC will be followed for the duration of stay.

At discharge or day 30, whichever comes first, a final assessment containing the same items as at Day 1, Dose 1 will be performed to generate a final measure of vitals and. Day 60 will be an outpatient follow-up visit, measuring vitals only and completing an adverse event inventory with all (living) subjects.

| ***Window*** | ***(prior* to *first dosing)*** | ***+*/*- 2 hours*** | **+/- *1 day*** | **+/- 3 *days*** |
|---|---|---|---|---|
| *Physical Exam including: vitals, (for all but follow-up) PaO2, FiO2, extrinsic PEEP, respirator settings as usually recorded in ICU* | *X* | *X q.i.d.* | *X* | *X* |
| *Hematology* | *X* | *X daily* | *X* | |
| *CBC, differential* | | | | |
| *Biochemistry* | *X* | *X daily* or *more frequently as recommended by attending physician* | *X* | |
| *Serum creatinine, BUN, sodium, potassium, chloride, total bilirubin, alkaline phosphatase, AST, ALT, LDH, total protein, calcium, phosphate, urinary myoglobin, GGT, albumin, random glucose, calculated creatinine clearance, CPK* | | | | |
| *Arterial blood gases* | *X* | *X daily* or *more frequently as recommended by attending physician* | | |
| *Coaqulation* | *X* | *As recommended by attending physician* | *As recommended by attending physician* | |
| *PT or INR, PTT* | | | | |
| *Pharmacokinetics* | | *[Sampling from blood daily blood draws]* | | |
| *Furosemide or placebo administration* | | *X q.i.d.* | | |
| *Adverse events* | X | *X twice daily on Day 1; daily Days 2-10* | *X weekly or as reported* | X |

### EXAMPLE 2: Furosemide anti-inflammatory activity

Various biologics have been proposed for use in addressing COVID-19 immuno-inlfammatory pathogenesis (e.g., tocilizumab, sarilumab, and adalimumab). Despite the obvious clinical utility of suchbiologics for many disorders, their specific applicability to COVID-19 is hampered by various issues: they target only one of the multiple cytokines implicated in COVID-19's immunopathy; if administered systemically, they can predispose patients to secondary infections or other toxicities, such as hepatoxicity; and they may be expensive to mass produce and distribute. Therefore, they are of limited utility in the context of a global pandemic. Furosemide was studied as a small molecule alternative having a broad-spectrum anti-inflammatory mechanism of action targeting cytokines of innate immunity, low toxicity, an excellent safety profile, and good chemically stability, and which can be easily stored and administered.

### Materials and Methods

### Inflammation activation on RAW264. 7 macrophage

RAW264.7 cells were purchased from ATCC and maintained in Dulbecco's Modified Eagle's Medium (DMEM) containing foetal bovine serum (FBS) at a final concentration of 10%. RAW264.7 cells were seeded in 12-well plates at 0.25 × 10⁶ cells/well seeding density, one day before experiments. To activate the cells, cell culture medium was changed to a lipopolysaccharide (LPS) and Interferon γ (IFNγ) containing medium with dimethyl sulfoxide (DMSO) or furosemide at required concentrations, followed by 24 h incubation at 37 °C; conditioned media and lysates were harvested for analysis.

### Inflammation activation on THP-1 monocytic cells

THP-1 cells (ATCC) were maintained in RPMI 1,640 medium supplemented with 2-mercaptoethanol at a final concentration of 0.05 mM and FBS at a final concentration of 10%. THP-1 cells were seeded in each well of a 12-well tissue culture plate at a density 0.5 × 10⁶ cells/mL, one day before experiments. THP-1 monocytes were differentiated by 150 nM PMA (phorbol 12-myristate 13-acetate) for 24 h. Cells were then treated with LPS+IFNγ with DMSO or furosemide at the required concentration, followed by 48 h incubation; conditioned media and lysates were collected for analysis.

### Nitric oxide production by Griess assay

Nitric oxide production from the conditioned media of RAW264.7 and SIM-A9 cell cultures was examined by a Griess assay. Conditioned medium and sulfanilamide were mixed in a microwell plate to form a transient diazonium salt. Then, *N*-naphtyl-ethylenediamine was added to all wells to form a stable azo compound by incubating for 5-10 min in the dark at room temperature. The absorbance was measured between 520 nm and 550 nm. The concentration of NO production was quantified by being plotted against a standard curve.

### Enzyme-linked immunosorbent assay

Cytokines were quantified using ELISA kits following the manufacturer's instructions. Briefly, the high-binding plates were coated at 100 µL/well with diluted capture antibodies (1:250) at 4 °C overnight. The coated plates were then blocked with the diluent for 1 h before assay. Each sample was diluted accordingly and added to the plates for a 2 h incubation period at room temperature. Plates were then washed with 250 µL/well PBS with 0.05% Tween-20 and incubated with detection antibodies (1:250 in assay diluent) for 1 h at room temperature. After another washing step, 1:250 diluted avidin-HRP (horseradish peroxidase) was added and incubated for 30 min. Next, 100 µL TMB-substrate (3,3',5,5'-tetramethylbenzidine) was added and the plate was incubated in the dark until the signal was sufficiently developed. The final concentration of TMB substrate solution was 200 µg/mL. The reaction was stopped with 50 µL of 2 N sulfuric acid. Absorbance was measured at 450 nm with a correction of 570 nm using a plate reader.

### Western blotting

Cells were washed twice with ice-cold PBS and harvested in RIPA buffer supplemented with a protease inhibitor cocktail. The whole-cell extracts were then centrifuged at 22,000×g for 20 min at 4 °C to remove cell debris. Protein concentrations were quantified using a Micro BCA protein assay kit. The absorbance was measured at 595 nm using a microplate reader. Equal amounts of cellular protein were separated by sodium dodecyl sulfate-polyacrylamide gel electrophoresis (SDS-PAGE) and transferred onto polyvinylidene difluoride (PVDF) membranes at 100 V for 90 min. The SDS-PAGE was performed with 10% polyacrylamide gel for iNOS and 12.5% for pro-IL-1B. The membranes were blocked for 1 h in Tris-buffered saline (TBS), pH 7.4, with 0.1% Tween-20 (TBS-T) containing 10% skim milk. The membrane blot was then incubated overnight at 4 °C with primary antibodies against iNOS (1:1,000), IL-1β (1:1,000), actin (1:5,000) and GAPDH (1:5,000) in TBS-T containing 5% skim milk. The membrane was washed with TBS-T 3 × 10 mins and incubated with goat anti-rabbit IgG-horseradish peroxidase (1:5,000) for 1 hour. After the washing step, the immunoblotting was visualized by chemiluminescence HRP-substrate.

### Flow cytometry

Cells were harvested and re-suspended with staining buffer. Cells were stained with antibody (1: 100) by incubating at 4 °C for 30 min in the dark. Stained cells were centrifuged, and the supernatant was discarded. The cell pellets were then re-suspended in cell flow buffer, transferred to FACS tubes and analyzed by flow cytometry within 48 h. To all cells in the experiment, Fc blocker was added.

### Inflammation activation on SIM-A9 cells

SIM-A9 (ATCC) cells were maintained in Dulbecco's modified eagle medium: nutrient mixture F-12 (DMEM-F12) with 10% foetal bovine serum, 5% horse-serum and antibiotic-antimycotic. SIM-A9 cells were seeded 24 h before the experiment. Culturing medium was replaced with DMEM-F12 medium containing 5% FBS + 2.5% horse serum with required LPS concentration (final volume was one mL/well). The conditioned media and lysates were harvested for cytokine and cell marker examination.

### Statistical analysis

Data are presented as mean ± SD or ±SEM. Statistical analysis was performed with GraphPad Prism software version 6.01c, applying a two-tailed unpaired t-test. Ap-value of >0.05 was considered significant.

### Results

To pre-clinically evaluate furosemide as a putative COVID-19 therapeutic, a series of *in vitro* efficacy assessments was performed to investigate its anti-inflammatory properties. First, furosemide was investigated to demonstrate that it could reduce the release of pro-inflammatory cytokines induced by LPS in macrophage cell line. RAW264.7 macrophages were stimulated with LPS in the presence or absence of furosemide and the levels of TNF-α and NO were measured from the conditioned media using ELISA and Griess assay, respectively. The results in Figs. 1A and 1B show that LPS induces the production of NO and TNF-α, indicating macrophage polarization to an M1 pro-inflammatory phenotype.

When cells were treated with LPS in the presence of 25 µM of furosemide, the production of NO and TNF-α significantly decreased. To further investigate the reduction of NO production by furosemide, we determined the expression level of inducible nitric oxide synthase (iNOS) which produces NO in response to inflammatory stimulations. Therefore, we stimulated RAW264.7 macrophages with LPS and IFNγ. The expression of iNOS was significantly induced by stimulation with LPS and IFNy, as shown by the Western blot results in Fig. 1C. We found that furosemide was able to suppress the expression of iNOS during LPS and IFNγ induced stimulation. Densitometry analysis showed that normalized iNOS/GAPDH ratio was reduced from 1 to 0.88 by furosemide.

LPS is recognized by the cell surface pattem-recognition receptors such as the toll-like receptor 4 protein (TLR4) and triggers downstream signaling pathways. LPS induces expression of the pro-inflammatory cytokine IFNγ. IFNγ is known to increase TLR4 expression which may then promote the response to LPS stimulation. The effect of furosemide on LPS-induced TLR4 expression in RAW264.7 macrophage cells was investigated using flow cytometry. As shown in Fig. 2, LPS increased the TLR4+ cell population significantly, whereas IL-4, an anti-inflammatory cytokine, barely induced TLR expression from the cells. Interestingly, furosemide completely blocked LPS-induced TLR4 expression, suggesting the possible involvement of furosemide in the LPS- or IFNy-induced inflammation.

As the next stage of macrophage activation, the activity of furosemide on the expression of IL-1β was studied. IL-1β plays a key role in modulating inflammatory response as a downstream pro-inflammatory marker of the TLR4 signaling pathway by using differentiated THP-1 monocytes. The expression of IL-1β precursor protein (pro-IL-1β) from THP-1 macrophage cells was studied using Western blot analysis. Furosemide, as shown in Fig. 3, significantly decreases the expression of pro-IL-1β in differentiated THP-1 cells, demonstrating yet another inflammatory cytokine targeted by furosemide. To further explore the effect of furosemide on different macrophages, it was tested on SIM-A9 cells. SIM-A9 macrophages were stimulated with LPS and the release of pro-inflammatory markers, such as NO, IL-6 and TNF-α, was studied. The results in Fig. 3 show that LPS induced the production of NO, IL-6 and TNF-α from SIM-A9 cells. Similar to RAW264.7 cell results, furosemide significantly reduced the production of all these pro-inflammatory markers from SIM-A9 cells as well.

Three different macrophage cell lines were tested to study the effects of furosemide on pro-inflammatory markers. Furosemide consistently reduced pro-inflammatory markers such as NO production, secretion of IL-6, TNF-α and expression of pro-IL-1β from different macrophage cell lines, implying that furosemide has broad inhibitory activity against pro-inflammatory cytokines.

Then, furosemide was evaluated to see if it exhibits any effects on anti-inflammatory cytokines. The levels of anti-inflammatory cytokines from the conditioned medium of differentiated THP-1 cells were measured after 48 h of stimulation with LPS and IFNγ by Multiplex assay using flow cytometry. Furosemide induces the expression of IL-4, interleukin-1 receptor antagonist (IL-1RA) and arginase, which are anti-inflammatory markers, suggesting the polarization of THP-1 macrophage to an M2 phenotype (Fig. 4).

Together with the experiments discussed above, these results show that furosemide inhibits the expression of M1 pro-inflammatory markers and promotes the expression of M2 anti-inflammatory markers. Thus, furosemide is a broad-spectrum anti-inflammatory drug candidate targeting multiple cytokines.

### Discussion

The pathogenic mechanisms of COVID-19 morbidity and mortality are diverse, though immuno-inflammatory contributions are likely a central player. It is now appreciated that COVID-19 afflicted individuals with major respiratory symptoms have pathologically elevated levels of pro-inflammatory cytokines including IL-6, IL-8 and TNF- 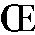 ± (Conti et al., 2020; Mehta et al., 2020; Qin et al., 2020; Huang et al., 2020). A logical therapeutic approach to the management of COVID-19 thus includes a need to modulate immunotoxicity.

Tryptophan and its metabolites, particularly via the indoleamine-2,3-dioxygenase initiated pathway, have a previously described role as endogenous modulators of innate immunity. Of these various metabolites, 3-HAA has been identified to exhibit a significant anti-inflammatory ability to suppress inflammation mediated via multiple pro-inflammatory interleukin cytokines, including IL-1β, IL-6, IL-8 and TNF-α (Lee et al., 2013). This motivated our search for an anthranilate-based 3-HAA-like agent from a library of known drugs; furosemide emerged as a possible candidate from this search.

In this Example, as part of its pre-clinical evaluation, furosemide's anti-inflammatory activity on multiple macrophage cell lines involved in innate immunity was studied. As pattern recognition receptors, TLRs contribute to the recognition of the molecules that are commonly shared by pathogens, such as LPS and viral nucleotides (Alexopoulou et al., 2001). The activation of TLRs triggers downstream signaling through the MyD88-dependent pathway and eventually induces the activation of nuclear factor-κB (NF-κB) (Oeckinghaus, Hayden & Ghosh, 2011). NF-κB has been shown to play an important role in coronavirus infections. For instance, NF-κB activation was identified in the lungs of SARS-CoV infected mice and triggered the production of pro-inflammatory cytokines, such as TNF-α (DeDiego et al., 2014). However, the mechanism of SARS-CoV pathogenesis is debated. An *in vitro* study suggested the nucleocapsid protein of SARS-CoV was crucial in the pathogenesis, although this correlation is cell-specific (Liao et al., 2005). On the other hand, SARS-CoV lacking the envelope protein (E protein) attenuated NF-κB activation and associated pro-inflammatory responses (DeDiego et al., 2014). The pandemic outbreak of COVID-19 is caused by the infection of SARS-CoV-2 that shares 79.5% identity to SARS-CoV (Guo et al., 2020). To investigate the potential use of furosemide as a therapy for COVID-19, *in vitro* assays were developed using LPS as exogenous stimulation to induce inflammatory responses. LPS-induced TLRs activation is well studied: it interacts with TLRs and triggers NF-κB activation followed by pro-inflammatory responses (Lu, Yeh & Ohashi, 2008). These *in vitro* assays were used to ascertain if LPS induces a similar "cytokine storm" as SARS-CoV-2 infection and if furosemide inhibits the production of pro-inflammatory cytokines or promotes the secretion of anti-inflammatory cytokine products.

Broadly conceptualized, stimulated macrophages can be polarized to either an M1 pro-inflammatory phenotype or an M2 anti-inflammatory phenotype. These studies investigated if furosemide inhibits the production of pro-inflammatory cytokines (M1) or promotes the secretion of anti-inflammatory cytokines (M2) using various macrophage cell lines including RAW264.7, THP-1 and SIM-A9. Upon stimulation, these cell lines initiated an immune response by producing cellular stress signals and secreting pro-inflammatory cytokines. These pro-inflammatory markers were reduced upon treatment with furosemide, indicating that furosemide suppresses the M1 polarization, including NO, IL-6 and TNF-α. More importantly, these study results demonstrated furosemide promotes the production of anti-inflammatory cytokine products (IL-1RA, arginase), indicating M2 polarization. All these results strongly support the use of furosemide as an immunomodulating agent for such disease conditions in which the inflammatory burden of patients increases suddenly. It also indicates that furosemide can be used for treating COVID-19 in which the sudden increase of pro-inflammatory cytokines is part of the disease pathogenesis.

Furosemide is a small molecule with a molecular weight of 330.75 g/mol and relatively low lipophilicity (logP = 2.03) (Hardman, Goodman & Gilman, 2001). Although the drug has low water solubility at pH 7, furosemide can be formulated in weakly basic buffer solution (pH 8) to achieve 10 mg/mL solutions suitable for intravenous administration. Due to the presence of a primary sulfonamide and carboxylic acid group, furosemide is highly bound to albumin with a human plasma protein binding value of 98.6 ± 0.4%. The drug has a very low volume of distribution (V_{D} = 0.13 ± 0.06 L/kg) and a relatively short half-life of 1.3 ± 0.8 h (Hardman, Goodman & Gilman, 2001). These pharmacokinetic parameters along with high plasma protein binding equates to low tissue distribution with furosemide being retained with the blood. Alveolar macrophages are front line innate immune cells, playing a crucial role in the maintenance of lung homeostasis and lung tissue defense through various immune responses. Tissue-resident alveolar macrophages are derived from the foetal liver and populate the alveoli shortly after birth. These macrophages are self-renewing and persist over the life span. However, exposure to environmental challenges and injury induces recruitment of monocyte-derived alveolar macrophages from circulating monocytes. The intrinsic molecular properties of furosemide are ideal for targeting macrophages recruited from the blood stream prior to their tissue distribution.

Furosemide exhibits a large therapeutic window and is listed on the WHO's List of "Essential Medicines"; it is readily available worldwide, is easily manufactured, and has a long record of safety and efficacy when given orally or intravenously. More importantly, furosemide may also be administered safely by inhalation. More than 20 years ago, the concept of inhaled furosemide was explored as an approach to reduce dyspnea, primarily based on the rationale that edematous airway mast cells would be reduced in size following diuresis (Prandota, 2002). However, further investigations established that the mechanism of action was not related to local diuretic effects or engagement of the Na⁺/K⁺-ATP shuttle. More recent studies have reported reduction in pulmonary IL-6, IL-8 and TNF-α levels upon administering inhaled furosemide to patients with conditions including tachypnea (Armed Forces Hospital Pakistan, 2016; University of Cologne, 2012), bronchopulmonary dysplasia (University of North Carolina et al., 2015) and chronic lung disease (Beth Israel Deaconess Medical Center, 2014; McGill University, 2016; Oxford Brookes University, 2015). A 2018 double-blind, placebo-controlled trial by Grogono et al. (2018) evaluated inhaled nebulized furosemide (40 mg furosemide in 4 mL 0.9% saline) in healthy adults, demonstrating effective relief of experimentally induced air hunger during dyspnea after multiple dosing per day with no untoward effects. Therefore, the accumulated data indicate that furosemide is a cytokine-targeting anti-inflammatory, which can be safely administered by inhalation multiple times per day.

### References

- Alexopoulou L, Holt AC, Medzhitov R, Flavell RA. 2001. Nature 413(6857):732-738
- Armagan G, Turunc E, Kanit L, Yalcin A. 2012. Free Radical Research 46(6):726-739
- Armed Forces Hospital Pakistan. 2016. Role of salbutamol and furosemide in TTN.
- Beth Israel Deaconess Medical Center. 2014. Aerosol inhalation treatment for dyspnea-patients.
- Boyce EG, Rogan EL, Vyas D, Prasad N, Mai Y. 2018. Annals of Pharmacotherapy 52(8):780-791
- Conti P, Ronconi G, Caraffa A, Gallenga C, Ross R, Frydas I, Kritas S. 2020. Journal of Biological Regulators and Homeostatic Agents 34(2):1
- Cortegiani A, Ingoglia G, Ippolito M, Giarratano A, Einav S. 2020. Journal of Critical Care 57:279-283
- DeDiego ML, Nieto-Torres JL, Regla-Nava JA, Jimenez-Guarde√±o JM, Fernandez-Delgado R, Fett C, Casta√±o-Rodriguez C, Perlman S, Enjuanes L. 2014. Journal of Virology 88(2):913-924
- Furst DE, Schiff MH, Fleischmann RM, Strand V, Birbara CA, Compagnone D, Fischkoff SA, Chartash EK. 2003. Journal of Rheumatology 30(12):2563-2571
- Grogono JC, Butler C, Izadi H, Moosavi SH. 2018. Respiratory Research 19(1):181-194
- Guo Y-R, Cao Q-D, Hong Z-S, Tan Y-Y, Chen S-D, Jin H-J, Tan K-S, Wang D-Y, Yan Y. 2020. Military Medical Research 7(1):11
- Hardman JG, Goodman LS, Gilman AG. 2001. Goodman & Gilman's the pharmacological basis of therapeutics. New York: McGraw-Hill Medical Publications.
- Hosseinimehr SJ, Nobakht R, Ghasemi A, Pourfallah TA. 2015. Radiation Oncology Journal 33(3):256-260
- Huang C, Wang Y, Li X, Ren L, Zhao J, Hu Y, Zhng L, Fan G, Xu J, Gu X+19 more. 2020. Lancet 395(10223):497-506
- Hung C-M, Peng C-K, Wu C-P, Huang K-L. 2018. Biochemical Pharmacology156:60-67
- Inokuchi R, Aoki A, Aoki Y, Yahagi N. 2014. Critical Care 18(6):621-626
- Lee W-S, Lee S-M, Kim M-K, Park S-G, Choi I-W, Choi I, Joo Y-D, Park S-J, KangS-W, Seo S-K. 2013. International Immunopharmacology 17(3):721-726
- Liao Q-J, Ye L-B, Timani KA, Zeng Y-C, She Y-L, Ye L, Wu Z-H. 2005. Acta Biochimica et Biophysica Sinica 37(9):607-612
- Lu Y-C, Yeh W-C, Ohashi PS. 2008. Cytokine 42(2):145-151
- McGill University. 2016. Inhaled nebulized furosemide & physical activity-related breathlessness.
- Mehta P, McAuley DF, Brown M, Sanchez E, Tattersall RS, Manson JJ. 2020. Lancet 395(10229):1033-1034
- National Cancer Institute Naples. 2020. Tocilizumab in COVID-19 Pneumonia (TOCIVID-19)
- Oeckinghaus A, Hayden MS, Ghosh S. 2011. Nature Immunology 12(8):695-708
- Oxford Brookes University. 2015. Specificity of dyspnoea relief with inhaled furosemide.
- O'Connor BJ, Chung KF, Chen-Worsdell YM, Fuller RW, Barnes PJ. 1991. American Review of Respiratory Disease 143(6):1329-1333
- Peking University First Hospital. 2020. Favipiravir combined with tocilizumab in the treatment of corona virus disease 2019.
- Prandota J. 2002. American Journal of Therapeutics 9(4):317-328
- Qin C, Zhou L, Hu Z, Zhang S, Yang S, Tao Y, Xie C, Ma K, Shang K, Wang W+1 more. 2020. Clinical Infectious Diseases ciaa248
- Regeneron Pharmaceuticals, Sanofi. 2020. Evaluation of the efficacy and safety of sarilumab in hospitalized patients with COVID-19.
- Sebba A. 2008. American Journal of Health-System Pharmacy 65(15):1413-1418
- Tongji Hospital, Hubei Xinhua Hospital, Wuhan No.1 Hospital, Wuhan central hospital. 2020. Tocilizumab vs CRRT in management of cytokine release syndrome (CRS) in COVID-19.
- University of Cologne. 2012. Trial on treatment with inhaled furosemide of preterm and term neonates with transient tachypnoea.
- University of North Carolina, Chapel Hill, Duke University, Eunice Kennedy Shriver National Institute of Child Health and Human Development, Human Development, The Emmes Company LLC. 2015. Safety of furosemide in premature infants at risk of bronchopulmonary dysplasia (BPD)
- Waskiw-Ford M, Wu A, Mainra A, Marchand N, Alhuzaim A, Bourbeau J, SmithBM, Jensen D. 2018. Frontiers in Physiology 9:86
- Xu X, Han M, Li T, Sun W, Wang D, Fu B. 2020. Proceedings of the National Academy of Sciences of the United States of America 117(20):10970-10975
- Yeo CT, O'Connor BJ, Chen-Worsdell M, Bames PJ, Chung KF. 1992. European Respiratory Journal 5(10):1184-1188
- Yuengsrigul A, Chin TW, Nussbaum E. 1999. Annals of Allergy, Asthma & Immunology 83(6):559-566

## Claims

1. Furosemide, or a pharmaceutically acceptable salt, solvate or hydrate thereof, for use in the treatment of a condition associated with coronavirus infection, wherein the condition is acute respiratory distress, lung inflammation, systemic inflammation, cytokine storm, or a combination thereof.

2. The furosemide, or a pharmaceutically acceptable salt, solvate or hydrate thereof, for use according to claim 1, wherein the furosemide, or a pharmaceutically acceptable salt, solvate or hydrate thereof, is formulated for administration via inhalation.

3. The furosemide, or a pharmaceutically acceptable salt, solvate or hydrate thereof, for use according to claim 2, wherein the furosemide, or a pharmaceutically acceptable salt, solvate or hydrate thereof, is formulated for administration via nebulization.

4. The furosemide, or a pharmaceutically acceptable salt, solvate or hydrate thereof, for use according to claim 2, wherein the furosemide, or a pharmaceutically acceptable salt, solvate or hydrate thereof, is formulated for administration via metered dose inhalation.

5. The furosemide, or a pharmaceutically acceptable salt, solvate or hydrate thereof, for use according to claim 2, wherein the furosemide, or a pharmaceutically acceptable salt, solvate or hydrate thereof, is formulated for administration via dry powder inhalation.

6. The furosemide, or a pharmaceutically acceptable salt, solvate or hydrate thereof, for use according to claim 1, wherein the furosemide, or a pharmaceutically acceptable salt, solvate or hydrate thereof, is formulated for intravenous administration.

7. The furosemide, or a pharmaceutically acceptable salt, solvate or hydrate thereof, for use according to claim 1, wherein the furosemide, or a pharmaceutically acceptable salt, solvate or hydrate thereof, is formulated for oral administration.

8. The furosemide, or a pharmaceutically acceptable salt, solvate or hydrate thereof, for use according to any one of claims 1 to 7, wherein the coronavirus is SARS-CoV-2.

9. The furosemide, or a pharmaceutically acceptable salt, solvate or hydrate thereof, for use according to any one of any one of claims 1 to 8, which is formulated for administration at a daily dose of between about 20 mg and about 300 mg.

10. The furosemide, or a pharmaceutically acceptable salt, solvate or hydrate thereof, for use according to claim 9, wherein the daily dose is between about 40 mg and about 200 mg.

11. The furosemide, or a pharmaceutically acceptable salt, solvate or hydrate thereof, for use according to claim 10, wherein the daily dose is about 200 mg, about 180 mg, about 160 mg, about 140 mg, about 120 mg, about 100 mg, about 80 mg, about 60 mg or about 40 mg.

12. The furosemide, or a pharmaceutically acceptable salt, solvate or hydrate thereof, for use according to any one of claims 1 to 11, which is formulated for administration once per day, or twice per day, or three times per day, or four times per day.

13. The furosemide, or a pharmaceutically acceptable salt, solvate or hydrate thereof, for use according to any one of claims 1 to 12, wherein the furosemide, or the pharmaceutically acceptable salt, solvate or hydrate thereof, is effective to reduce the levels of pro-inflammatory cytokines in a subject to levels lower than the levels of pro-inflammatory cytokines present prior to said use.

## Patentansprüche

1. Furosemid oder ein pharmazeutisch annehmbares Salz, Solvat oder Hydrat davon zur Verwendung bei der Behandlung eines Zustands, der mit einer Coronavirusinfektion assoziiert ist, wobei der Zustand akute Atemnot, Lungenentzündung, systemische Inflammation, Zytokinsturm oder eine Kombination davon ist.

2. Das Furosemid oder ein pharmazeutisch annehmbares Salz, Solvat oder Hydrat davon zur Verwendung gemäß Anspruch 1, wobei das Furosemid oder ein pharmazeutisch annehmbares Salz, Solvat oder Hydrat davon formuliert ist für die Verabreichung durch Inhalation.

3. Das Furosemid oder ein pharmazeutisch annehmbares Salz, Solvat oder Hydrat davon zur Verwendung gemäß Anspruch 2, wobei das Furosemid oder ein pharmazeutisch annehmbares Salz, Solvat oder Hydrat davon formuliert ist für die Verabreichung durch Zerstäubung.

4. Das Furosemid oder ein pharmazeutisch annehmbares Salz, Solvat oder Hydrat davon zur Verwendung gemäß Anspruch 2, wobei das Furosemid oder ein pharmazeutisch annehmbares Salz, Solvat oder Hydrat davon formuliert ist für die Verabreichung durch gemessene-Dosis-Inhalation.

5. Das Furosemid oder ein pharmazeutisch annehmbares Salz, Solvat oder Hydrat davon zur Verwendung gemäß Anspruch 2, wobei das Furosemid oder ein pharmazeutisch annehmbares Salz, Solvat oder Hydrat davon formuliert ist für die Verabreichung durch Trockenpulverinhalation.

6. Das Furosemid oder ein pharmazeutisch annehmbares Salz, Solvat oder Hydrat davon zur Verwendung gemäß Anspruch 1, wobei das Furosemid oder ein pharmazeutisch annehmbares Salz, Solvat oder Hydrat davon formuliert ist für intravenöse Verabreichung.

7. Das Furosemid oder ein pharmazeutisch annehmbares Salz, Solvat oder Hydrat davon zur Verwendung gemäß Anspruch 1, wobei das Furosemid oder ein pharmazeutisch annehmbares Salz, Solvat oder Hydrat davon formuliert ist für orale Verabreichung.

8. Das Furosemid oder ein pharmazeutisch annehmbares Salz, Solvat oder Hydrat davon zur Verwendung gemäß einem der Ansprüche 1 bis 7, wobei das Coronavirus SARS-CoV-2 ist.

9. Das Furosemid oder ein pharmazeutisch annehmbares Salz, Solvat oder Hydrat davon zur Verwendung gemäß einem der Ansprüche 1 bis 8, welches formuliert ist für die Verabreichung bei einer täglichen Dosis zwischen etwa 20 mg und etwa 300 mg.

10. Das Furosemid oder ein pharmazeutisch annehmbares Salz, Solvat oder Hydrat davon zur Verwendung gemäß Anspruch 9, wobei die tägliche Dosis zwischen etwa 40 mg und etwa 200 mg beträgt.

11. Das Furosemid oder ein pharmazeutisch annehmbares Salz, Solvat oder Hydrat davon zur Verwendung gemäß Anspruch 10, wobei die tägliche Dosis etwa 200 mg, etwa 180 mg, etwa 160 mg, etwa 140 mg, etwa 120 mg, etwa 100 mg, etwa 80 mg, etwa 60 mg oder etwa 40 mg beträgt.

12. Das Furosemid oder ein pharmazeutisch annehmbares Salz, Solvat oder Hydrat davon zur Verwendung gemäß einem der Ansprüche 1 bis 11, welches formuliert ist für die Verabreichung einmal pro Tag, oder zweimal pro Tag, oder dreimal pro Tag, oder viermal pro Tag.

13. Das Furosemid oder ein pharmazeutisch annehmbares Salz, Solvat oder Hydrat davon zur Verwendung gemäß einem der Ansprüche 1 bis 12, wobei das Furosemid oder das pharmazeutisch zulässige Salz, Solvat oder Hydrat davon wirksam ist, die Spiegel von proinflammatorischen Zytokinen in einem Subjekt zu reduzieren auf Spiegel, die niedriger sind als die Spiegel von proinflammatorischen Zytokinen vor dieser Verwendung.

## Revendications

1. Furosémide, ou sel, solvate ou hydrate pharmaceutiquement acceptable de celui-ci, pour une utilisation dans le traitement d'un état pathologique associé à une infection à coronavirus, l'état pathologique étant une détresse respiratoire aiguë, une inflammation du poumon, une inflammation systémique, un choc cytokinique, ou une combinaison de ceux-ci.

2. Furosémide, ou sel, solvate ou hydrate pharmaceutiquement acceptable de celui-ci, pour une utilisation selon la revendication 1, dans lequel le furosémide, ou un sel, solvate ou hydrate pharmaceutiquement acceptable de celui-ci, est formulé pour administration par inhalation.

3. Furosémide, ou sel, solvate ou hydrate pharmaceutiquement acceptable de celui-ci, pour une utilisation selon la revendication 2, dans lequel le furosémide, ou un sel, solvate ou hydrate pharmaceutiquement acceptable de celui-ci, est formulé pour administration par nébulisation.

4. Furosémide, ou sel, solvate ou hydrate pharmaceutiquement acceptable de celui-ci, pour une utilisation selon la revendication 2, dans lequel le furosémide, ou un sel, solvate ou hydrate pharmaceutiquement acceptable de celui-ci, est formulé pour administration par inhalation par aérosol-doseur.

5. Furosémide, ou sel, solvate ou hydrate pharmaceutiquement acceptable de celui-ci, pour une utilisation selon la revendication 2, dans lequel le furosémide, ou un sel, solvate ou hydrate pharmaceutiquement acceptable de celui-ci, est formulé pour administration par inhalation de poudre sèche.

6. Furosémide, ou sel, solvate ou hydrate pharmaceutiquement acceptable de celui-ci, pour une utilisation selon la revendication 1, dans lequel le furosémide, ou un sel, solvate ou hydrate pharmaceutiquement acceptable de celui-ci, est formulé pour administration intraveineuse.

7. Furosémide, ou sel, solvate ou hydrate pharmaceutiquement acceptable de celui-ci, pour une utilisation selon la revendication 1, dans lequel le furosémide, ou un sel, solvate ou hydrate pharmaceutiquement acceptable de celui-ci, est formulé pour administration orale.

8. Furosémide, ou sel, solvate ou hydrate pharmaceutiquement acceptable de celui-ci, pour une utilisation selon l'une des revendications 1 à 7, dans lequel le coronavirus est le SARS-CoV-2.

9. Furosémide, ou sel, solvate ou hydrate pharmaceutiquement acceptable de celui-ci, pour une utilisation selon l'une des revendications 1 à 8, formulé pour administration à une dose journalière comprise entre environ 20 mg et environ 300 mg.

10. Furosémide, ou sel, solvate ou hydrate pharmaceutiquement acceptable de celui-ci, pour une utilisation selon la revendication 9, dans lequel la dose journalière est comprise entre environ 40 mg et environ 200 mg.

11. Furosémide, ou sel, solvate ou hydrate pharmaceutiquement acceptable de celui-ci, pour une utilisation selon la revendication 10, dans lequel la dose journalière est d'environ 200 mg, d'environ 180 mg, d'environ 160 mg, d'environ 140 mg, d'environ 120 mg, d'environ 100 mg, d'environ 80 mg, d'environ 60 mg ou d'environ 40 mg.

12. Furosémide, ou sel, solvate ou hydrate pharmaceutiquement acceptable de celui-ci, pour une utilisation selon l'une des revendications 1 à 11, formulé pour administration une fois par jour, ou deux fois par jour, ou trois fois par jour, ou quatre fois par jour.

13. Furosémide, ou sel, solvate ou hydrate pharmaceutiquement acceptable de celui-ci, pour une utilisation selon l'une des revendications 1 à 12, dans lequel le furosémide, ou un sel, solvate ou hydrate pharmaceutiquement acceptable de celui-ci est efficace pour réduire les niveaux de cytokines pro-inflammatoires chez un sujet jusqu'à des niveaux inférieurs aux niveaux de cytokines pro-inflammatoires présents avant ladite utilisation.
